# EUROPEAN PATENT APPLICATION

(11) **EP 4 527 906 A1**
(43) Date of publication of application: **26.03.2025**
(21) Application number: 23306553.1
(22) Date of filing: 20.09.2023
(51) Int. Cl.: C09K 15/06, C07B 63/04, C09K 15/08, C07D 493/04, C09K 15/04

(54) **OXIDATION RESISTANT COMPOSITIONS OF DINAHYDROHEXITOLS CONTAINING BENZOPHENONE OR ITS DERIVATIVES**

(71) Applicant: ROQUETTE FRERES, 62136 Lestrem (FR)
(72) Inventor: SAINT-LOUP, René, 59160 LOMME (FR); VANBESIEN, Théodore, 59280 Armentières (FR)
(74) Representative: Plasseraud IP

(57) **Abstract**

One object of the present application is a composition comprising chemical compound having at least one heterocycle, and at least one antioxidant chosen from antioxidants having at least two aromatic cycles. Particularly the chemical compound is chosen from dianhydrohexitols, most particularly is isosorbide. Said composition show improved resistance to oxidation, and lower yellow coloration over storage and after thermal treatment. These compositions are useful for the preparation of polymers comprising isosorbide as a comonomer.

## Description

### Technical Field

The invention pertains to the field of use of antioxidants on dianhydrohexitols or derivatives of dianhydrohexitols to protect said dianhydrohexitols or derivatives thereof from degradation reactions initiated by oxidation.

### Background Art

The main issue with dianhydrohexitols or their derivatives, specifically their esters, is their chemical instability, and may arise anywhere and anytime starting from their synthesis.

The production of a monoanhydrohexitol or dianhydrohexitol usually requires a polyol as the starting material. This polyol may have been previously obtained by the hydrogenation of a sugar.

The process of production of dianhydrohexitols usually involves the following main steps: 1) synthesis of dianhydrohexitols, 2) purification of said dianhydrohexitols, 3) shaping of said purified dianhydrohexitols. In the case of derivatives of dianhydrohexitols such as ethers or esters thereof, further similar steps are required: 4) synthesis of derivative of dianhydrohexitol from dianhydrohexitol obtained previously, 5) purification of dianhydrohexitol derivative, 6) shaping of purified dianhydrohexitols. Furthermore, at the end of each process, the products must be stored over periods of time ranging from weeks to months to years.

The process of production in general, and in particular the purification steps involving high temperatures, for instance in the range 100-250°C, usually requires special care to avoid thermal degradation of said compounds. Furthermore, their long-term storage at ambient temperature in containers can also induce slow degradation.

Several means have been designed in the prior art, usually relying on the addition of a compound or mix of compounds able to protect the dianhydrohexitols or their derivatives from the thermal degradation or the oxidation. Those compounds are called "stabilizers" or "stabilizing agent".

Among the prior art, several compounds have been identified with stabilizing properties for dianhydrohexitols or their derivatives, such as those listed in EP1,446,373:
- reducing agents, and in particular boron- or aluminum-based compounds such as sodium borohydride (NaBH4) or lithium aluminohydride (LiAIH4);
- phosphorus-based compounds such as phosphine or phosphite;
- antioxidant agents, and in particular nitrogen-based compounds, in particular amines, aromatic or not, containing or not otherwise at least one alcohol function, such as hydroxylamine, morpholine, and their derivatives, or such as monoethanolamine, diethanolamine (DEA), triethanolamine (TEA), these three being the object of EP2,991 ,991 from the applicant, or mixtures thereof, or such as cyclic amines in WO2017/095016;
- aromatic compounds, nitrogenous or not, containing or not at least one alcohol function, such as hydroquinone, phenol, tocopherols and their respective derivatives;
- antioxidant compounds based on phosphorus or sulfur such as phosphites, phosphonites, sulphites, salts of thiodipropionic acid esters and mixtures thereof;
- antacids;
- metal deactivating agents, such as complexing or chelating agents of metals of natural origin;
- products authorized as food additives, in particular those qualified as anti-oxidants, acidity regulators or sequestrants within the meaning of European regulations, such as ascorbic acid (vitamin C), erythorbic acid, lactic acid, citric acid, gallic acid, tocopherols, derivatives (in particular salts) of all these products, BHT, butylhydroxyanisole (BHA) and any mixtures of these products.

Recently, continuing its research to obtain compositions of dianhydrohexitols ever more stable, the Applicant came to the conclusion that oxidation was a preponderant phenomenon; In other words, the stability of said compositions is primarily governed by their resistance to oxidation. In doing so, the Applicant succeeded in identifying a particular class of chemical compounds, particularly effective in improving this oxidation stability: gallic acid esters, also called gallates, as disclosed in the patent application FR2108221 filed by the applicant.

### Technical Problem

The latest stabilizers identified by the applicant, namely the esters of gallic acid, have a shortcoming: they are not stable to temperature, as their ester function is hydrolyzed in presence of water at temperatures above 60-100°C. This hydrolysis leads to formation of organic acids, which react to give colored compounds when further submitted to temperatures above 150-200°C. This limits the scope of use of such stabilizers. During the process of production of isosorbide, they cannot be added before any process step involving a temperature above 60-100°C in presence of water, for instance distillation.

Indeed, the applicant tried to implement the stabilization of isosorbide solution with gallate esters at high temperatures, like the ones typically occurring in distillation of such solutions, which is from around 100°C to around 250°C, more usually around 200-250°C, and obtained unacceptable yellow coloration levels due to the organic acids created from thermal degradation of the gallate esters.

An easy way to circumvent this problem for the production of isosorbide would be to ensure that the addition of the stabilizer is performed at the very end of the process of production of isosorbide, where no more heat treatments occur. Somehow, this would limit the scope of action of such stabilizer to the very end of the production process, whereas it could be helpful to be able to add the stabilizer into earlier production steps in order to protect the isosorbide from oxidation as early as possible in its purification and/or shaping process steps.

So, there is a need for an antioxidant stabilizer that withstands, i.e. that retains its chemical structure, when exposed to thermal treatments. Furthermore, such stabilization should be effective for isosorbide under any state possible, liquid or solid form such as crystal, pellets or flakes, and should not affect the coloration, the smell and the physico-chemical properties of isosorbide.

### Brief Description of Drawings

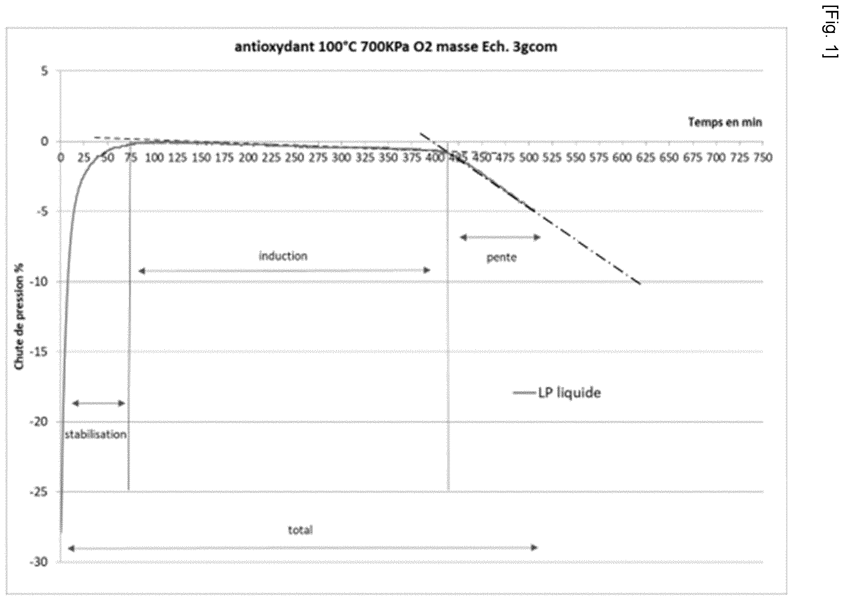

Other features, details and advantages will be shown in the following detailed description and on the figures, on which: **Fig. 1**
[Fig. 1] is a graph representative of the methodology of the PetroOxy test useful for determining the induction time;

### Description of Embodiments

Embodiments of the objects of the present application are the following.

Embodiment 1: composition comprising:
- at least one chemical compound having at least one heterocycle,
- and at least one antioxidant of formula (I): 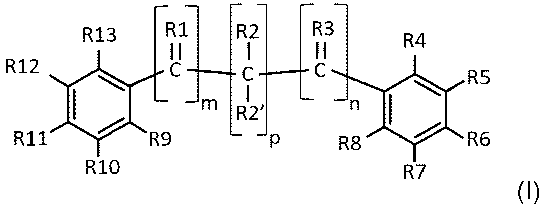 wherein:
   - R1 and R3 are selected from oxygen, CH2, saturated or unsaturated alkyl groups comprising 2 to 10 carbon atoms, or a combination thereof,
   - R2 and R2' are selected from hydrogen, CH3, saturated or unsaturated alkyl groups comprising 2 to 10 carbon atoms, aromatic cycles, or a combination thereof,
   - R4 to R13 are selected from H, OH, CₓH₂ₓ₊₁, O-C_{y}H_{2y+1}, x and y being integers ranging from 1 to 10,
   - m, n and p are integers in the range 0 to 10, and when p = 0 then m and/or n is greater than or equal to 1, or when both m and n are equal to 0 then p is greater than or equal to 1.

Embodiment 2: composition according to embodiment 1, wherein the antioxidant is of formula (II): 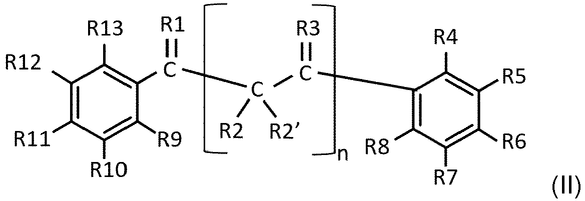 wherein:
- R1 and R3 are selected from oxygen, CH2, saturated or unsaturated alkyl groups comprising 2 to 10 carbon atoms, or a combination thereof,
- R2 and R2' are selected from hydrogen, CH3, saturated or unsaturated alkyl groups comprising 2 to 10 carbon atoms, aromatic cycles, or a combination thereof,
- R4 to R13 are selected from H, OH, CₓH₂ₓ₊₁, O-C_{y}H_{2y+1}, x and y being integers ranging from 1 to 10,
- n is an integer in the range 0 to 10.

Embodiment 3: composition according to embodiment 1, wherein the antioxidant is of formula (III): 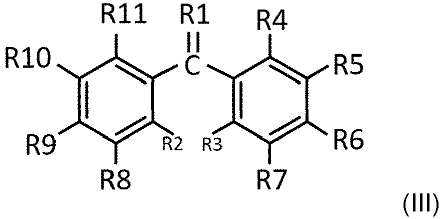 wherein:
- R1 is selected from oxygen, CH2, saturated or unsaturated alkyl groups comprising 2 to 10 carbon atoms,
- R2 to R11 are selected from H, OH, CₓH₂ₓ₊₁, O-C_{y}H_{2y+1}, x and y being integers ranging from 1 to 10.

Embodiment 4: composition according to embodiment 3, wherein the antioxidant is benzophenone compound of formula (IV): 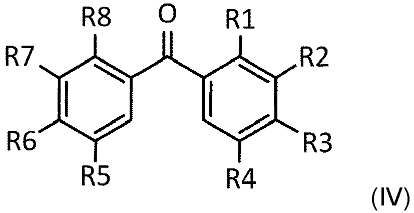 wherein R1 to R8 are selected from H, OH, CH3, O-CH3.

Embodiment 5: composition according to embodiment 4, wherein the benzophenone compounds of formula (III) are compounds of formula (V) to (VIII):

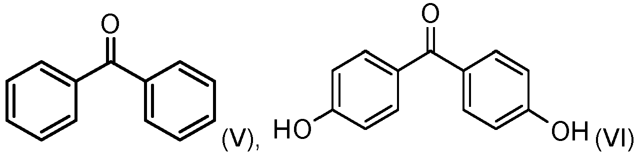

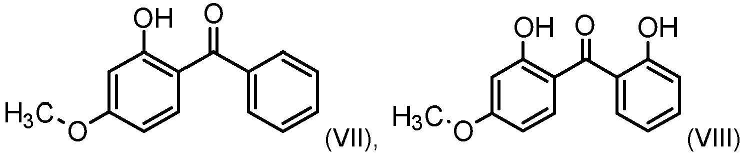

Embodiment 6: Composition according to embodiment 4, wherein the benzophenone compound of formula (III) is 2-hydroxy-4-methoxy benzophenone of formula (VII).

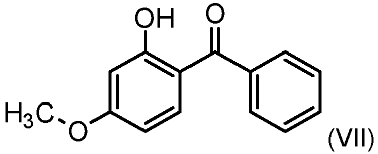

Embodiment 7: composition according to embodiment 1, wherein the antioxidant is of formula (IX): 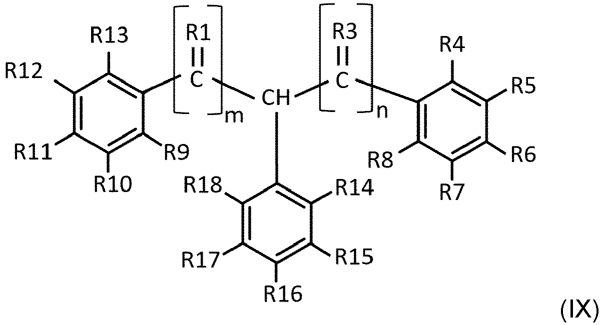 wherein:
- R1 and R3 are selected from oxygen, CH2, saturated or unsaturated alkyl groups comprising 2 to 10 carbon atoms, or a combination thereof,
- R4 to R18 are selected from H, OH, CₓH₂ₓ₊₁, O-C_{y}H_{2y+1}, x and y being integers ranging from 1 to 10,
- m and n are integers in the range 0 to 10.

Embodiment 8: composition according to any one of the preceding embodiments, wherein the content of the antioxidant in the composition is equal to or less than 1 000 ppm with respect to the weight of the organic chemical compound, in particular equal to or less than 500 ppm, or equal to or less than 400 ppm, or equal to or less than 300 ppm, or equal to or less than 200 ppm, or equal to or less than 100 ppm.

Embodiment 9: composition according to any one of the preceding embodiments, wherein the composition further comprises at least one stabilizing compound selected from gallic acid, gallic acid esters, phosphate compounds, amines, or cyclic amines.

Embodiment 10: composition according to embodiment 9, wherein the stabilizing compound is selected from ethyl gallate, propyl gallate, disodium phosphate, diethanolamine, triethanolamine, tetramethylpiperidine, tetramethylpipéridinone diazabicyclo-nonene, diazabicyclo-undecene or mixtures thereof.

Embodiment 11: composition according to any one of the preceding embodiments, wherein the composition is a liquid composition or a solid composition.

Embodiment 12: composition according to embodiment 11, wherein the content in chemical compound having at least one heterocycle in said composition is at least 50 wt%, in particular at least 60 wt%, or at least 70 wt%, or at least 80 wt%, or at least 90 wt%, or at least 95 wt%, or at least 98 wt%, or at least 99 wt%.

Embodiment 13: composition according to embodiment 11, wherein the solid composition is a crystalline solid composition, a semi-crystalline solid composition or an amorphous solid composition.

Embodiment 14: composition according to embodiment 13, wherein the solid composition is in the form of crystals, pellets or flakes.

Embodiment 15: composition according to embodiment 11, wherein the composition has a water content of at most 50 wt%, in particular of at most 40 wt%, or at most 30 wt%, or at most 20 wt%, or at most 10 wt%, at most 5 wt%, or of at most 1 wt%, or of at most 0,5 wt%, or of at most 0,1 wt%, or of at most 0,01 wt%.

Embodiment 16: composition according to any one of the preceding embodiments, wherein the chemical compound having at least one heterocycle is a heterocyclic compound or a derivative thereof.

Embodiment 17: composition according to embodiment 16, wherein the heterocyclic compound results from an intramolecular dehydration of a hydrogenated sugar, or is a derivative of a product of an intramolecular dehydration of a hydrogenated sugar.

Embodiment 18: composition according to embodiment 17, wherein the hydrogenated sugar is selected from sorbitol, mannitol, or xylitol, or mixtures thereof, in particular sorbitol.

Embodiment 19: composition according to embodiment 17, wherein the heterocyclic compound is selected from monoanhydrohexitols, dianhydrohexitols, or mixtures thereof, in particular from isosorbide, isoidide, or isomannide, or mixtures thereof, more particularly is isosorbide.

Embodiment 20: composition according to embodiment 17, wherein the derivative of an intramolecular dehydration product is selected from monoesters, diesters, monoethers, diethers, or combination thereof, in particular from monoesters or diesters, more particularly from diesters.

Embodiment 21: composition according to embodiment 20, wherein the derivative is selected from monoesters of dianhydrohexitols, diesters of dianhydrohexitols, monoethers of dianhydrohexitols, diethers of dianhydrohexitols, or a combination thereof, in particular from monoesters or diesters of dianhydrohexitols, more particularly from diesters of dianhydrohexitols.

Embodiment 22: composition according to any one of the preceding embodiments, wherein it exhibits an induction time greater than or equal to 3.8 hours, in particular greater than or equal to 4.0 hours, more particularly greater than or equal to 4.5 hours, most particularly greater than or equal to 5.0 hours, as measured according to the "PetroOxy test" at 120°C and 7 bars of oxygen.

Embodiment 23: composition according to the preceding embodiment 22, wherein it exhibits a yellow coloration index equal to or less than 13.0 YID, in particular equal to or less than 5.0 YID, more particularly equal to or less than 3.0 YID, most particularly equal to or less than 2.5 YID.

Embodiment 24: composition according to preceding embodiments 22 or 23, wherein it exhibits a yellow coloration index after thermal treatment test, equal to or less than 50.0 YID, in particular equal to or less than 10.0 YID, more particularly equal to or less than 8.0 YID, most particularly equal to or less than 6.5 YID.

Embodiment 25: process for preparing a composition as described in any of embodiments 1 to 24, comprising:
a) an intramolecular dehydration step of a hydrogenated sugar to obtain a dehydration product in a crude reaction mixture,
b) optionally a step of derivatization of the dehydration product obtained in step a) to obtain a derivative of the dehydration product in a crude reaction mixture,
c) optionally a step of distillation of the crude reaction mixtures obtained in step a) and/or in step b) to obtain a distillate enriched with the dehydration product or the derivative thereof,
d) optionally a step of purification of the crude reaction mixtures obtained in step a) and/or in step b), and/or of the distillate obtained in step c), to obtain a purified dehydration product,
e) optionally a step of shaping the crude reaction mixtures obtained in step a) and/or in step b), and/or shaping of the distillate obtained in step c), and/or shaping of the purified dehydration product obtained in step d), to obtain a shaped dehydration product,
f) a step of adding at least one antioxidant of formula (I) to the crude reaction mixtures obtained in step a) and/or in step b), and/or to the distillate obtained in step c), and/or to the purified dehydration product obtained in step d), and/or to the shaped dehydration product obtained in step e), 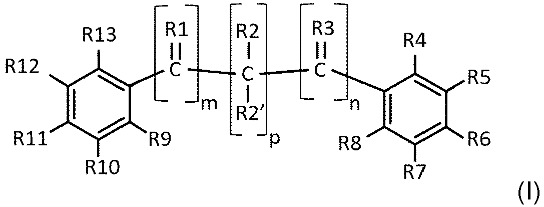 wherein:
   - R1 and R3 are selected from oxygen, CH2, saturated or unsaturated alkyl groups comprising 2 to 10 carbon atoms, or a combination thereof,
   - R2 and R2' are selected from hydrogen, CH3, saturated or unsaturated alkyl groups comprising 2 to 10 carbon atoms, aromatic cycles, or a combination thereof,
   - R4 to R13 are selected from H, OH, CₓH₂ₓ₊₁, O-C_{y}H_{2y+1}, x and y being integers ranging from 1 to 10,
   - m, n and p are integers in the range 0 to 10, and when p = 0 then m and/or n is greater than or equal to 1, or when both m and n are equal to 0 then p is greater than or equal to 1.

Embodiment 26: process according to embodiment 25, wherein the antioxidant is of formula (II): 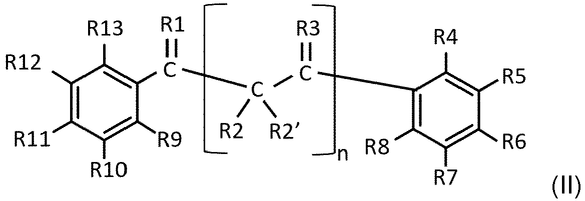 wherein:
- R1 and R3 are selected from oxygen, CH2, saturated or unsaturated alkyl groups comprising 2 to 10 carbon atoms, or a combination thereof,
- R2 and R2' are selected from hydrogen, CH3, saturated or unsaturated alkyl groups comprising 2 to 10 carbon atoms, aromatic cycles, or a combination thereof,
- R4 to R13 are selected from H, OH, CₓH₂ₓ₊₁, O-C_{y}H_{2y+1}, x and y being integers ranging from 1 to 10,
- n is an integer in the range 0 to 10.

Embodiment 27: process according to embodiment 25, wherein the antioxidant is of formula (III): 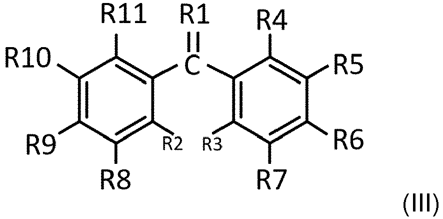 wherein:
- R1 is selected from oxygen, CH2, saturated or unsaturated alkyl groups comprising 2 to 10 carbon atoms,
- R2 to R11 are selected from H, OH, CₓH₂ₓ₊₁, O-C_{y}H_{2y+1}, x and y being integers ranging from 1 to 10.

Embodiment 28: process according to embodiment 27, wherein the antioxidant is benzophenone compound of formula (IV): 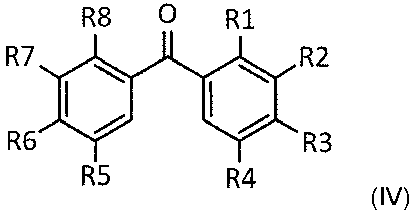 wherein R1 to R8 are selected from H, OH, CH3, O-CH3.

Embodiment 29: process according to embodiment 28, wherein the benzophenone compounds of formula (III) are compounds of formula (V) to (VIII):

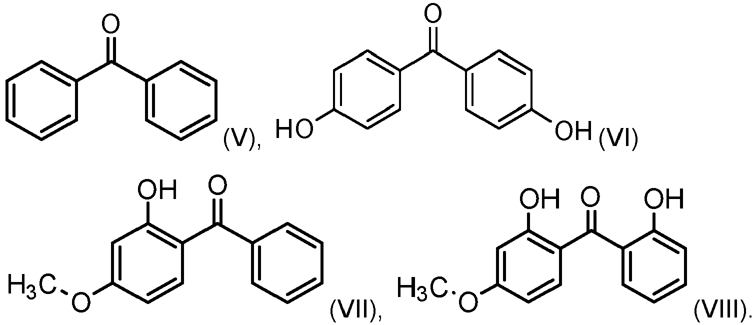

Embodiment 30: process according to embodiment 28, wherein the benzophenone compound of formula (III) is 2-hydroxy-4-methoxy benzophenone of formula (VII).

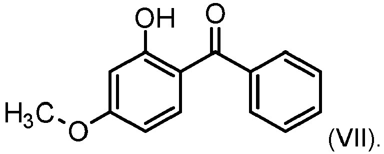

Embodiment 31: process according to embodiment 25, wherein the antioxidant is of formula (IX): 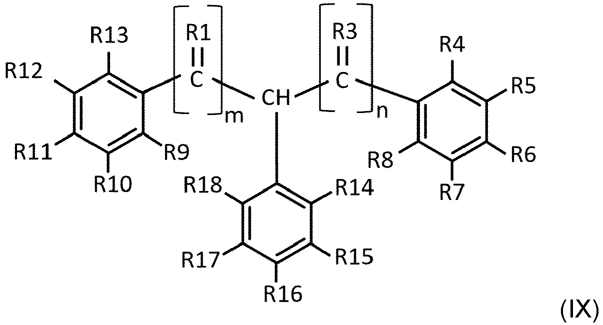 wherein:
- R1 and R3 are selected from oxygen, CH2, saturated or unsaturated alkyl groups comprising 2 to 10 carbon atoms, or a combination thereof,
- R4 to R18 are selected from H, OH, CₓH₂ₓ₊₁, O-C_{y}H_{2y+1}, x and y being integers ranging from 1 to 10,
- m and n are integers in the range 0 to 10.

Embodiment 32: process according to embodiment 25, wherein it comprises:
a) an intramolecular dehydration step of a hydrogenated sugar to obtain a dehydration product,
b) a subsequent distillation step of the crude reaction mixture obtained in step a) to obtain a distillate enriched with the dehydration product,
c) a purification step of the distillate enriched with the dehydration product obtained in step b), in particular chosen from a crystallization step, an activated charcoal treatment step, or a demineralization on resins step, or a combination thereof, to obtain a purified dehydration product,
d) a step of adding at least one antioxidant according to formula (I) to the purified dehydration product obtained in step c) to obtain a composition,
e) a step of shaping the composition obtained after step d), in particular a step of concentrating, or a step of crystallization, or a step of transformation into flakes or into pellets.

Embodiment 33: use of an antioxidant of formula (I) 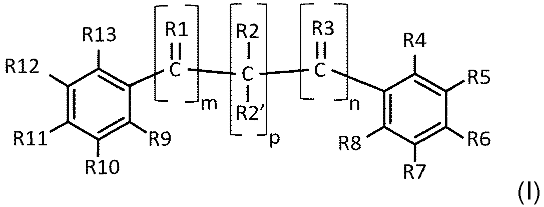 in a composition comprising at least one chemical compound having at least one heterocycle, for preventing or reducing the chemical degradation of said dehydration product or derivative thereof,
wherein:
- R1 and R3 are selected from oxygen, CH2, saturated or unsaturated alkyl groups comprising 2 to 10 carbon atoms, or a combination thereof,
- R2 and R2' are selected from hydrogen, CH3, saturated or unsaturated alkyl groups comprising 2 to 10 carbon atoms, aromatic cycles, or a combination thereof,
- R4 to R13 are selected from H, OH, CₓH₂ₓ₊₁, O-C_{y}H_{2y+1}, x and y being integers ranging from 1 to 10,
- m, n and p are integers in the range 0 to 10, and when p = 0 then m and/or n is greater than or equal to 1, or when both m and n are equal to 0 then p is greater than or equal to 1..

Embodiment 34: use according to embodiment 33, wherein the antioxidant is of formula (II): 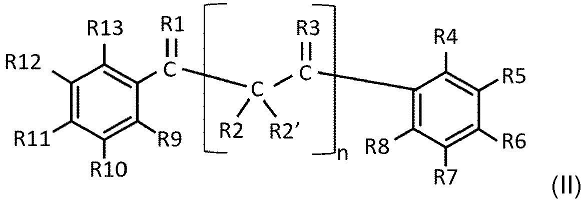 wherein:
- R1 and R3 are selected from oxygen, CH2, saturated or unsaturated alkyl groups comprising 2 to 10 carbon atoms, or a combination thereof,
- R2 and R2' are selected from hydrogen, CH3, saturated or unsaturated alkyl groups comprising 2 to 10 carbon atoms, aromatic cycles, or a combination thereof,
- R4 to R13 are selected from H, OH, CₓH₂ₓ₊₁, O-C_{y}H_{2y+1}, x and y being integers ranging from 1 to 10,
- n is an integer in the range 0 to 10.

Embodiment 35: use according to embodiment 33, wherein the antioxidant is of formula (III): 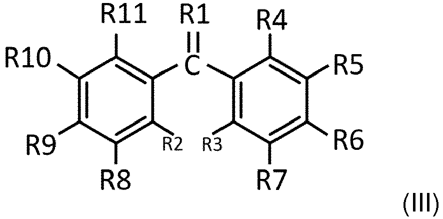 wherein:
- R1 is selected from oxygen, CH2, saturated or unsaturated alkyl groups comprising 2 to 10 carbon atoms,
- R2 to R11 are selected from H, OH, CₓH₂ₓ₊₁, O-C_{y}H_{2y+1}, x and y being integers ranging from 1 to 10.

Embodiment 36: use according to embodiment 35, wherein the antioxidant is benzophenone compound of formula (IV): 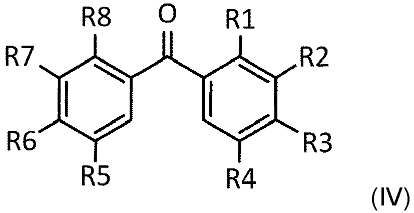 wherein R1 to R8 are selected from H, OH, CH3, O-CH3.

Embodiment 37: use according to embodiment 36, wherein the benzophenone compounds of formula (III) are compounds of formula (V) to (VIII):

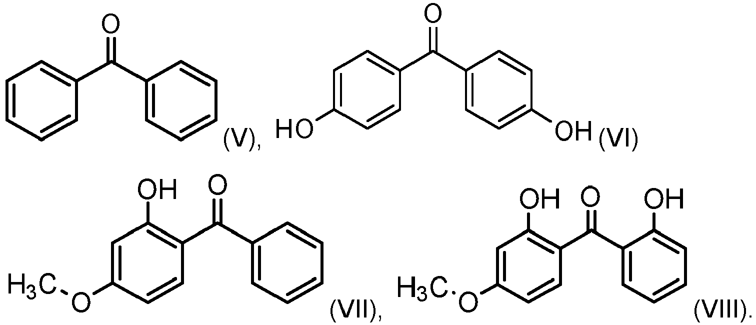

Embodiment 38: use according to embodiment 36, wherein the benzophenone compound of formula (III) is 2-hydroxy-4-methoxy benzophenone of formula (VII).

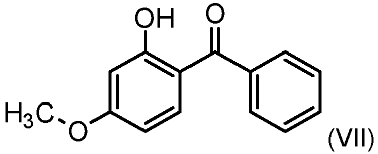

Embodiment 39: use according to embodiment 33, wherein the antioxidant is of formula (IX): 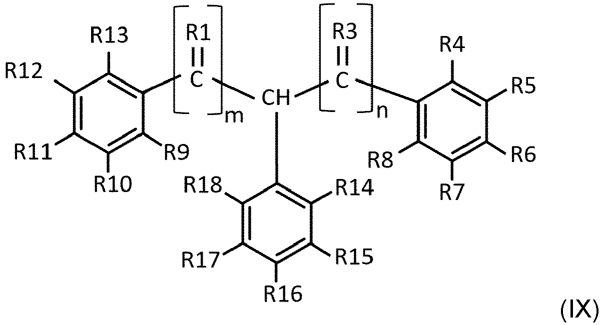 wherein:
- R1 and R3 are selected from oxygen, CH2, saturated or unsaturated alkyl groups comprising 2 to 10 carbon atoms, or a combination thereof,
- R4 to R18 are selected from H, OH, CₓH₂ₓ₊₁, O-C_{y}H_{2y+1}, x and y being integers ranging from 1 to 10,
- m and n are integers in the range 0 to 10.

Embodiment 40: use according to any one of the embodiments 33 to 39, wherein the content of the antioxidant in the composition is equal to or less than 1 000 ppm with respect to the weight of the organic chemical compound, in particular equal to or less than 500 ppm, or equal to or less than 400 ppm, or equal to or less than 300 ppm, or equal to or less than 200 ppm, or equal to or less than 100 ppm.

Embodiment 41: use according to any one of the embodiments 33 to 40, wherein the composition further comprises at least one stabilizing compound selected from gallic acid, gallic acid esters, phosphate compounds, amines, or cyclic amines.

Embodiment 42: use according to embodiment 41, wherein the stabilizing compound is selected from ethyl gallate, propyl gallate, disodium phosphate, diethanolamine, triethanolamine, tetramethylpiperidine, tetramethylpipéridinone diazabicyclo-nonene, diazabicyclo-undecene or mixtures thereof.

Embodiment 43: use according to any one of the embodiments 33 to 42, wherein the composition is a liquid composition or a solid composition.

Embodiment 44: use according to embodiment 43, wherein the content in chemical compound having at least one heterocycle in said composition is at least 50 wt%, in particular at least 60 wt%, or at least 70 wt%, or at least 80 wt%, or at least 90 wt%, or at least 95 wt%, or at least 98 wt%, or at least 99 wt%.

Embodiment 45: use according to embodiment 43, wherein the solid composition is a crystalline solid composition, a semi-crystalline solid composition or an amorphous solid composition.

Embodiment 46: use according to embodiment 45, wherein the solid composition is in the form of crystals, pellets or flakes.

Embodiment 47: use according to embodiment 43, wherein the composition has a water content of at most 50 wt%, in particular of at most 40 wt%, or at most 30 wt%, or at most 20 wt%, or at most 10 wt%, at most 5 wt%, or of at most 1 wt%, or of at most 0,5 wt%, or of at most 0,1 wt%, or of at most 0,01 wt%.

Embodiment 48: use according to any one of the embodiments 33 to 47, wherein the chemical compound having at least one heterocycle is a heterocyclic compound or a derivative thereof.

Embodiment 49: use according to embodiment 48, wherein the heterocyclic compound results from an intramolecular dehydration of a hydrogenated sugar, or is a derivative of a product of an intramolecular dehydration of a hydrogenated sugar.

Embodiment 50: use according to embodiment 49, wherein the hydrogenated sugar is selected from sorbitol, mannitol, or xylitol, or mixtures thereof, in particular sorbitol.

Embodiment 51: use according to embodiment 49, wherein the heterocyclic compound is selected from monoanhydrohexitols, dianhydrohexitols, or mixtures thereof, in particular from isosorbide, isoidide, or isomannide, or mixtures thereof, more particularly is isosorbide.

Embodiment 52: use according to embodiment 49, wherein the derivative of an intramolecular dehydration product is selected from monoesters, diesters, monoethers, diethers, or combination thereof, in particular from monoesters or diesters, more particularly from diesters.

Embodiment 53: use according to embodiment 52, wherein the derivative is selected from monoesters of dianhydrohexitols, diesters of dianhydrohexitols, monoethers of dianhydrohexitols, diethers of dianhydrohexitols, or a combination thereof, in particular from monoesters or diesters of dianhydrohexitols, more particularly from diesters of dianhydrohexitols.

Embodiment 54: use according to any one of the embodiments 33 to 53, wherein the composition exhibits an induction time greater than or equal to 3.8 hours, in particular greater than or equal to 4.0 hours, more particularly greater than or equal to 4.5 hours, most particularly greater than or equal to 5.0 hours, as measured according to the "PetroOxy test" at 120°C and 7 bars of oxygen.

Embodiment 55: use according to the preceding embodiment 54, wherein the composition exhibits a yellow coloration index equal to or less than 13.0 YID, in particular equal to or less than 5.0 YID, more particularly equal to or less than 3.0 YID, most particularly equal to or less than 2.5 YID.

Embodiment 56: use according to any of the embodiments 54 or 55, wherein the composition exhibits a yellow coloration index after thermal treatment test, equal to or less than 50.0 YID, in particular equal to or less than 10.0 YID, more particularly equal to or less than 8.0 YID, most particularly equal to or less than 6.5 YID.

Embodiment 57: use of a composition according to any one of embodiments 1 to 24 for the preparation of products or mixtures, polymeric or not, biodegradable or not, for the chemical, pharmaceutical, cosmetic or food industries.

Embodiment 58: use according to embodiment 57, wherein the use is the preparation of polyesters or polycarbonates comprising said intramolecular dehydration product of a hydrogenated sugar as a co-monomer, in particular comprising isosorbide as a co-monomer:

### Detailed description

### Chemical compounds comprising at least one heterocycle

The chemical compound having at least one heterocycle is a substance comprising carbon and hydrogen, and at least one different chemical element chosen from oxygen, sulfur and nitrogen, which is part of the heterocycle. The heterocycle of said organic chemical compound is a cycle that has atoms of carbon and hydrogen, and atoms of at least one different element chosen from oxygen, sulfur and nitrogen, as its constituting members.

In one embodiment, the chemical compound having at least one heterocycle is an organic chemical compound consisting of atoms of carbon and of hydrogen, and of at least one different atom chosen from oxygen, sulfur and nitrogen.

In one embodiment, the organic chemical compound having at least one heterocycle consists of atoms of carbon, hydrogen and oxygen, and has two heterocycles consisting of four atoms of carbon and one atom of oxygen.

In one embodiment, the organic chemical compound is a product of an intramolecular dehydration of a hydrogenated sugar, or derivatives of said product. In particular, the sugar is chosen from pentoses or hexoses. More particularly, said product is a monoanhydrohexitol, dianhydrohexitol, or mixtures thereof.

### Heterocyclic compounds

A heterocyclic compound is a cyclic compound that consists of heterocycles, that is to say whose entire structure is made of heterocycles sharing some atoms as constituting members and does not have any alkyl groups connected to said heterocycles.

### Intramolecular dehydration product of a hydrogenated sugar

Sugars, such as pentoses or hexoses, may be hydrogenated to transform their aldehyde function into an alcohol function, thus forming a hydrogenated sugar, belonging to the family of polyols. Under appropriate chemical conditions, one hydroxyl function of the hydrogenated sugar can make a nucleophilic substitution on a carbon bearing hydroxyl groups of the very same hydrogenated sugar, thus forming an heterocycle within the hydrogenated sugar. This intramolecular reaction releases a molecule of water, which is why it is called an intramolecular dehydration reaction. Depending on the structure of the obtained hydrogenated sugar, a second intramolecular dehydration can be done, thus giving a compound with two heterocycles.

The polyol obtained from hexoses may then be transformed into heterocyclic compounds known "dianhydrohexitols" also called "isohexides". The dianhydrohexitols are the products of the double intramolecular dehydration of hexoses, thus having two heterocycles. The hexoses are C6 hydrogenated sugars, also called hexitols, such as sorbitol, mannitol and iditol. The intramolecular dehydration is usually carried out in acidic conditions at high temperatures of 120-200°C with a catalyst. In the present application, the term monoanhydrohexitol include the 1,4-sorbitan (1,4-anhydro-sorbitol), the iditan (1,4-monoanhydro-iditol), and the mannitans (2,5-anhydro-mannitol, 1,4-anhydro-mannitol); the term "dianhydrohexitols" includes isosorbide which is 1, 4 - 3,6- dianhydro-sorbitol, isomannide which is 1, 4 - 3,6-dianhydro-mannitol, and isoidide which is 1, 4 - 3,6-dianhydro-iditol, as well as mixtures of these products.

The products obtained during or after intramolecular dehydration of hydrogenated sugars can be a mixture of unreacted reactants, intermediate products and final products, said final products may be numerous and various according to chemical selectivity of the reaction, all of these being in a solvent, for instance water in the case of anhydrohexitols. The mixture of all these compounds and the solvent constitutes a "crude reaction mixture", either during the course of the reaction, or at the end of the reaction once the reaction is completed and stopped.

### Derivatives of product of intramolecular dehydration of hydrogenated sugar

Products of intramolecular dehydration of hydrogenated sugars comprise hydroxyl groups, either on the cycle formed by intramolecular dehydration, or on the remaining linear polyol part, that may be esterified or etherified. Derivatives according to the present application are the esters and ethers obtained by such an esterification or etherification. According to one embodiment, derivatization of the intramolecular dehydration products means the esterification or the etherification of said dehydration products, in particular the esterification.

In one embodiment, the derivatives are monoesters of dianhydrohexitols, diesters of dianhydrohexitols, or mixtures thereof. In particular, said monoesters or diesters have a saturated or unsaturated alkyl chain comprising from 2 to 22 atoms of carbon.

In one embodiment, the derivatives are monoethers of dianhydrohexitols, diether of dianhydrohexitols, or mixtures thereof. In particular, said monoethers or diethers have a saturated or unsaturated alkyl chain comprising from 2 to 22 atoms of carbon.

### Antioxidant

The antioxidant comprised in the composition according to the present application is an organic molecule able to suppress free radicals that may be generated by oxidation reactions within the composition according to the invention. This suppression allows to stop the degradation by two ways: the first one is by stopping the formation of hydrogen peroxide, and the second one is by stopping the chain reactions of any free radicals. These two actions prevent and stop the degradation of compounds having an heterocycle.

Dioxygen may become a free radical known as "superoxide", which may react with water to form hydrogen peroxide. Hydrogen peroxide may then oxide compounds having heterocycles, creating degradation products such as organic acids and alcohols. The antioxidant can react with superoxide free radicals to give dioxygen back, becoming itself a free radical. The resulting antioxidant bearing a free radical will dimerize, or even trimerize, with another free radical beared by another antioxidant, which will make all free radicals disappear.

The superoxide may also react with a first compound comprising a heterocycle, in particular with the oxygen contained in the heterocycle. This will open the heterocycle, and thus create a free radical on said first compound, which will be able to further react with the oxygen of a second compound comprising a heterocycle. This reaction will again open the heterocycle of said second compound, thus creating again another free radical on said second compound. This process may repeat again and again, and is called a free radical chain reaction, until all heterocycles are opened, that is to say when all compounds initially comprising heterocycles are chemically degraded. To avoid this, the free radical chain reaction must be stopped. This is what the antioxidant does: it reacts with a free radical formed from a compound comprising a heterocycle, transforming it in a non-free radical compound, and becoming itself a free radical. The resulting antioxidant bearing a free radical will dimerize, or even trimerize, with another free radical beared by another antioxidant, which will make all free radicals disappear.

The antioxidants useful for the composition according to the invention are organic molecules that comprise at least two aromatic cycles connected one to another by a carbon chain. Their general chemical formula is the following (I): 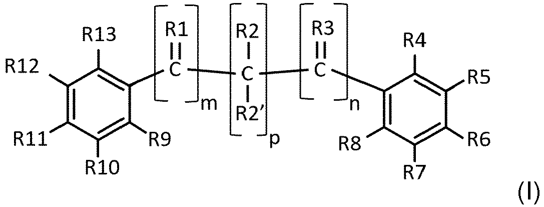 wherein:
- R1 and R3 are selected from oxygen, CH2, saturated or unsaturated alkyl groups comprising 2 to 10 carbon atoms, or a combination thereof,
- R2 and R2' are selected from hydrogen, CH3, saturated or unsaturated alkyl groups comprising 2 to 10 carbon atoms, aromatic cycles, or a combination thereof,
- R4 to R13 are selected from H, OH, CₓH₂ₓ₊₁, O-C_{y}H_{2y+1}, x and y being integers ranging from 1 to 10,
- m, n and p are integers in the range 0 to 10, and when p = 0 then m and/or n is greater than or equal to 1, or when both m and n are equal to 0 then p is greater than or equal to 1.

Antioxidants according to formula (I) can make chemical compounds having heterocycles, or aqueous solutions thereof, be resistant to oxidation, even at temperatures above 100°C, in particular above 150°C, more particularly above 200°C, and most particularly above 250°C. According to one embodiment, the antioxidant according to formula (I) can make heterocyclic compounds, in particular, heterocyclic compounds resulting from intramolecular dehydration of hydrogenated sugars, be resistant to oxidation, even at temperatures above 100°C, in particular above 150°C, more particularly above 200°C, and most particularly 250°C.

The substituents R4 to R13 linked to the aromatic cycles of antioxidant of formula (I) may be advantageously selected to enhance the antioxidant property of said antioxidant, and thus increase the resistance to oxidation of compounds having heterocycles, or aqueous solutions thereof. Without being bound to a theory, the applicant think that such selection may help stabilize the free radicals formed on the antioxidant and increase the dimerization of free radical antioxidants one with another, therefor reducing or eliminating the possibility for free radical antioxidants to react with compounds having heterocycles. In particular, the applicant found that the presence of one hydroxyl group and of one methoxy group on the same aromatic cycle greatly increase the antioxidant efficiency on aqueous compositions comprising chemical compound having at least one heterocycle, or a derivative of said chemical compound.

Antioxidants of formula (I) wherein at least one of R4 to R13 is a hydroxyl group, or wherein at least one of R4 to R13 is a hydroxyl group and at least one of R4 to R13 is a methoxy group, can make resistance to oxidation be from slightly increased to greatly increased, compared to antioxidant of formula (I) where all R4 to R13 are hydrogens. In particular, antioxidants of formula (I) wherein at least one of R4 to R13 is a hydroxyl group and at least one of R4 to R13 is a methoxy group exhibit a greatly increased antioxidant property, leading to greatly increased resistance to oxidation for the composition according to the invention. More particularly, antioxidants of formula (I) wherein at least one of R4 and/or R13 is a hydroxyl group and at least one of R6 and/or R11 is a methoxy group exhibit a greatly increased antioxidant property, leading to greatly increased resistance to oxidation for the composition according to the invention. For instance, the antioxidant 2-hydroxy-4-methoxy benzophenone of formula (VII) 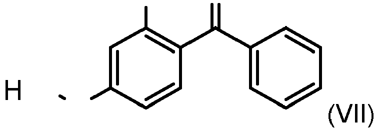 is able to make an isosorbide aqueous solution very resistant to oxidation, with induction times around 4,6 hours as measured by the PetroOxy test of the present application. Another example is the 2,2'-dihydroxy-4-methoxybenzophenone of formula (VIII) 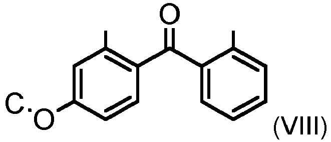 which gives an induction time of 5.2 hours.

### Resistance to oxidation

The composition according to the present application is resistant to oxidation. According to the present application, "resistant to oxidation" means that no chemical degradation of the chemical compound having at least one heterocycle occurs at temperatures ranging from 4°C to 25°C for a duration of at least six months. Said chemical degradation is the opening of the heterocycle, thus forming an organic acid and an alcohol, and other degradation products, which are yellow colored. This chemical degradation may therefore be characterized by the monitoring of the pH and of yellow coloration index of said composition over time. Due to the high level of resistance to oxidation now achieved with recently identified antioxidants, the applicant looked for and found a faster method, able to give results and show differences in resistance to oxidation in hours, not months. This is the "PetroOxy" method commonly used in the petrochemicals field, and applied for the first time by the Applicant to isosorbide solutions as disclosed in a previous patent application from the Applicant FR2108221.

The resistance of compositions according to the invention to oxidation may indeed be characterized by this measurement called the "PetroOxy test" which consists in measuring over time the pressure inside a cell loaded with a sample of composition exposed to an initial pressure of dioxygen and a constant temperature. The protocol of this test is described below after.

In one embodiment, "resistant to oxidation" means the induction time according to the PetroOxy test of the present application (which is at 120°C and 7 bars of oxygen), is greater than or equal to 3,8 hours. In a preferred embodiment, "resistant to oxidation" means the induction time is greater than or equal to 4.0 hours, in particular greater than or equal to 4.5 hours, more particularly greater than or equal to 5.0 hours, as measured according to the PetroOxy test of the present application, at 120°C and 7 bars of oxygen.

### Yellow coloration

The yellow coloration is indicative of two things inside the composition according to the present application: one is the inner coloration of the antioxidant; the other is the presence of degradation products generated by oxidation. Antioxidant according the formula (I) of the present application are inherently colored due to their aromatic cycles, and so are their degradation products by oxidation and the resulting dimers or trimers.

On the one hand, for some end uses, a yellow coloration due to the antioxidant is acceptable, because it will be masked by pigments. On the other hand, for other end uses of the composition according to the invention, the yellow coloration must be as low as possible both on the composition freshly obtained, or on the composition stored for several months, or even on the composition processed to produce an end use product. The yellow coloration after several months may be simulated by accelerated aging, which consists in a submitting a sample of the composition to a heat treatment, such as the "thermal treatment test" described below after.

The composition of the invention has a medium to very low yellow coloration, or is even deprived of yellow coloration. "Medium yellow coloration" means that it has a yellow coloration index from 20.0 YID to 13.1 YID. "Low yellow coloration" means that it has a yellow coloration index from 13.0 YID to 5.1 YID. A "very low yellow" coloration means that it has a yellow coloration index from 5.0 YID to 3.0 YID. "Deprived of yellow coloration" means that the yellow coloration index is equal to or less than 3.0 YID, in particular equal to or less than 2.5 YID.

In one embodiment, the composition according to the invention exhibit a high resistance to oxidation and a medium level of yellow coloration. In particular, in this embodiment, the high resistance to oxidation means an induction time greater than or equal to 5 hours, and the medium level of yellow coloration means a yellow coloration index from 20.0 YID to 13.1 YID, and a high yellow coloration index after thermal, which means a yellow coloration index after thermal treatment test according to the present application from 50.0 YID to 20.0 YID.

In another embodiment, the composition according to the present application exhibit both a good resistance to oxidation and is deprived of yellow coloration as measured according to the tests and methods below after. In particular in this embodiment, a good resistance to oxidation means an induction time from 4.0 hours to 4.9 hours, and deprived of yellow coloration means a yellow coloration index before thermal treatment lower than or equal to 3.0 YID, and a yellow coloration index after thermal treatment test lower than or equal to 10.0 YID.

### Additional stabilizing compounds

According to one embodiment, some additional stabilizing compounds known from the prior art may be added to the composition according to the invention, in order to provide further protection to chemical degradations, or strengthen the resistance to oxidation provided by the antioxidant according to the invention. These stabilizing compounds may be antioxidants that stabilize the composition by preventing or reducing oxidation reactions in a similar manner as the antioxidant of formula (I) according to the invention. They can also act by neutralizing the acidic degradation products, thereby preventing these from further degrading the chemical compound comprising at least one heterocycle.

These optional additional stabilizing compounds are selected from gallic acid, gallic acid esters, phosphate compounds, amines, cyclic amines, or mixtures thereof. In particular, they are selected from ethyl gallate, propyl gallate, disodium phosphate, diethanolamine, triethanolamine, tetramethylpiperidine, tetramethylpipéridinone diazabicyclo-nonene, diazabicyclo-undecene or mixtures thereof.

According to another embodiment, the composition of the invention is free of any additional stabilizing compounds.

### Process for preparing a composition according to the invention

The process according to the present invention produces a composition, of at least one compound having a heterocycle, which is resistant to oxidation, and which may further have a medium to very low yellow coloration, or even be deprived of yellow coloration. It does so thanks to the addition of an antioxidant of formula (I) after any of its steps a), b), c), d) or e): 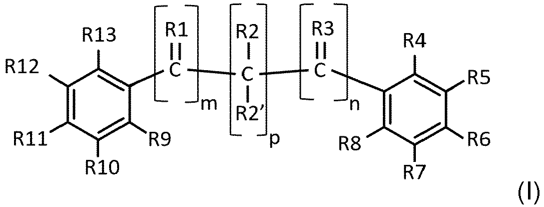 wherein:
- R1 and R3 are selected from oxygen, CH2, saturated or unsaturated alkyl groups comprising 2 to 10 carbon atoms, or a combination thereof,
- R2 and R2' are selected from hydrogen, CH3, saturated or unsaturated alkyl groups comprising 2 to 10 carbon atoms, aromatic cycles, or a combination thereof,
- R4 to R13 are selected from H, OH, CₓH₂ₓ₊₁, O-C_{y}H_{2y+1}, x and y being integers ranging from 1 to 10,
- m, n and p are integers in the range 0 to 10, and when p = 0 then m and/or n is greater than or equal to 1, or when both m and n are equal to 0 then p is greater than or equal to 1.

### Purification

The purification step d) of the process according to the invention removes impurities from the crude reaction mixtures obtained in step a) and/or step b), or from the distillate obtained in step c).

According to one embodiment, step d) consists of at least one purification means selected from the discoloration means and the ion exchange means.

By "means of discoloration" are meant in particular activated carbon in granular or powdery form and adsorption resins. For example, granular activated carbon such as CECA DC 50, powdery activated carbon such as NORIT SX+ and / or a resin such as DUOLITE XAD 761, MACRONET MN-600 or MACRONET MN-400 may be used. By means of "ion exchange" are meant in particular anionic resins, weak or strong, and cationic resins, weak or strong. For example, one can use, alone or in combination, a strong anionic resin such as AMBERLITE IRA 910 resin or a strong cationic resin such as PUROLITE C 150 S resin. The ion exchange medium may advantageously comprise at least one anionic resin and at least one cationic resin. Preferably, this means is composed of a mixed bed of anionic and cationic resin(s), or a succession of cationic resin(s) then anionic resin(s), or a succession of anionic resin(s) then cationic resin(s).

In particular, in step d) of the process according to the invention, the crude reaction mixtures obtained in step a) and/or b), or the distillate obtained in step c), are treated in any order by at least one activated carbon and at least one resin, ionic or nonionic. According to one embodiment, a first purification is performed by an activated carbon, followed by at least one resin; and then again by an activated carbon.

### Shaping

The shaping step e) of the process according to the invention modifies the physical state of any of the matters obtained in step a), b), c) or d), either by changing at least one characteristic of said physical state while keeping the physical state in the same category, which is one of liquid or solid, or changes the physical state into another physical state, for instance liquid into solid, solid into liquid.

In particular, the shaping encompasses the following physical states: solutions, in particular aqueous solutions; molten liquid; mass solid, in particular pellets or flakes; dispersed solid, in particular crystals.

In particular, the at least one characteristic of the physical state changed while keeping said physical state in same category are the dry matter content of a liquid, the water content of a solid, the size distribution of a dispersed solid, the crystallinity of a solid. Crystallinity means one of crystalline, semi-crystalline or amorphous.

The means to perform the shaping of step e) may be selected from concentration by evaporation, concentration by membrane filtration, vacuum drying, fluidized bed drying, horizontal cylindrical dryer, crystallization by solvent evaporation, crystallization by cooling, or molten state crystallization.

### "PetroOxy" test: method for measuring the induction time

The method hereafter is described for isosorbide as the chemical compound having at least one heterocycle, and is also applicable to any chemical compound having at least one heterocycle.

3 grams of a purified 50 wt% dry matter isosorbide solution in distilled water with or without the addition of antioxidant are weighed precisely in a glass cup and then placed in the hermetic enclosure of the measuring device "PetroOxy" model 13-3002 from Petrotest, now Anton Paar GmbH. The air present in the cell is purged 3 times at room temperature with pure oxygen via a pressurized cylinder (Alphagas, purity >99.5%). The temperature and working pressure of pure oxygen is then set at 120°C and 700 kPa (7 bars) of oxygen. The pressure is continuously monitored during the experiment. The decrease in pressure in the test chamber is due to oxygen consumption during oxidation reactions of the sample studied. The variation in relative pressure during the experiment makes it possible to determine the time after which the sample begins its degradation by oxidation, which is called induction time, also referred as "OIT", under the test conditions.

The induction time of the sample is determined as follows:

The relative pressure of oxygen consumption is given by the relation (Pmax -Pt)/Pmax. Pmax is the maximum pressure attained by the system during the measurement, corresponding to the maximum pressure at the beginning of time, and Pt is the pressure as a function of time when oxygen is consumed. The induction time of the sample is given by the inflection point of the oxygen consumption curve, i.e. the intersection of the two linear domains as shown on figure Fig.1.

The longer the induction time, the greater the oxidation resistance of the sample. To calculate precisely this parameter, a calculation method by linear regression between the two-line segments before and after the inflection point is performed.

### Method for measuring the yellow coloration index

The yellow coloration index, expressed in YID units, was measured using a Colorflex EZ from Hunterlab according to ASTM E313. The spectrophotometer is a sealed optics, 256-elements diode array, having a 400-700 nm spectral range and a high-resolution concave holographic grating. 55 grams of composition are placed at 65°C, in a 64 mm diameter cylindric cell including a ring and white disk cap. The coloration of the composition was measured as a liquid at 65°C.

### "Thermal treatment" test: method for determining the thermal stability of compositions to temperature

The method hereafter is described for isosorbide as the chemical compound having at least one heterocycle, and is also applicable to any chemical compound having at least one heterocycle.

The thermal stability of compositions of isosorbide, was studied according to the following protocol: 70 g of a 80 wt% solution of isosorbide in water was placed in a three-necked round flask and inerted with high purity nitrogen (purity >99.9%) during 10 minutes at a flowrate of around 2000 mL/min. The product was then heated at 245°C using an oil bath for 40 minutes under a 500 mL/min nitrogen flowrate. Then the product was cooled down to 80°C under the same nitrogen flowrate and yellow coloration measurement (YID value) was measured immediately.

Coloration before and after "thermal treatment" as well as their difference is related to the product thermal stability, i.e. to the sensitivity of the product to oxidation.

### Examples

### Example 1: preparation of an unstabilized isosorbide composition and study of its induction time and yellow index coloration.

An unstabilized isosorbide solution was prepared as follows:
In a double jacketed stirred reactor, we introduced 1 kg of a solution of sorbitol at 70 wt% dry matter (MS) marketed by the Applicant under the name NEOSORB 70 / 02, and 7 g of concentrated sulfuric acid. The mixture obtained was heated under vacuum (100mbars) for 5 hours so as to remove the water contained in the reaction medium and that from the dehydration reaction of sorbitol.

The reaction crude was then cooled to 100 ° C and neutralized with 11.4 g of a 50% sodium solution. The isosorbide composition was distilled under vacuum (pressure less than 50 mbar).

The isosorbide distillate, slightly colored (light yellow) was then dissolved in isopropanol at a temperature of 60 ° C, so as to obtain a homogeneous solution of 75 wt% dry matter. This solution was then cooled slowly, within 5 hours, to a temperature of 10°C. A recrystallized isosorbide primer was added at 40°C.

The crystals were then wrung out in a spinner and washed with isopropanol. After vacuum drying, the crystals were redissolved in water to obtain an aqueous solution of 50 wt% dry matter.

This solution was then percolated on a column of granular activated carbon (GC 12-40 at a relative speed of 0.5V/V/h (product volume/resin volume per hour). The discolored isosorbide composition obtained was passed at a rate of 2 V / V / h successively on a column of strong cationic resin (PUROLITE C150 S then on a strong anionic resin AMBERLITE IRA910. This solution was then treated with powdered activated carbon (NORIT SX+) at 20°C for one hour. The amount of coal used was 0.5% by weight relative to the dry weight of solution.

After filtration of the activated carbon, the isosorbide solution was recovered and stored at 4°C in high-density polyethylene containers in a dark room. It was analyzed according to the above methodology.

The induction time of isosorbide solution without additives is 3.5 h. This value will serve as a reference for determining the efficacy of antioxidants.

### Example 2: preparation of stabilized isosorbide solutions and study of their induction time and yellow coloration index

Various stabilized isosorbide solutions were prepared according to the following protocol:
1000 g of unstabilized isosorbide solution prepared in example 1 were introduced in flask under nitrogen. 50 mg of antioxidant were added to this isosorbide solution. Mixing with magnetic stirrer was carried out during 180 minutes to ensure homogenous blend between the isosorbide and the antioxidant. The content in antioxidant is thus equal to 100 ppm of dry matter of antioxidant with respect to dry matter of isosorbide. The stabilized isosorbide solution was then kept at 4 °C under nitrogen for storage.

Each stabilized isosorbide solution was characterized for its induction time according to the "PetroOxy test" and for its coloration index (YID) before and after thermal test. Results are shown in table 1.

**[Table 1]**

| Sample # | Antioxidant compound | Induction time (hours) | % versus no antioxidant |
|---|---|---|---|
| 1 | None | 3.5 | n.a. |
| 2.1 | Benzophenone | 3.8 | + 9 |
| 2.2 | 4,4'-dihydroxy-benzophenone | 3.9 | + 11 |
| 2.3 | 2-hydroxy-4-methoxybenzophenone | 4.6 | + 31 |
| 2.4 | 2,2'-dihydroxy-4-methoxybenzophenone | 5.2 | + 49 |

Results from table 1 show the four benzophenone compounds were able to increase the induction time of an isosorbide aqueous solution, which means these were able to delay the onset of the oxidation in such isosorbide solutions.

2,2'-dihydroxy-4-methoxy-benzophenone gave the highest increase in induction time, which means that it has the highest resistance against oxidation.

These results show that the oxidation of isosorbide solutions by oxygen may be delayed using benzophenone compounds. The momentum of the delay is surprisingly dependent on the nature of the substituents beared by the aromatic cycles, and vary in an unpredictable way.

### Example 3: resistance of stabilized isosorbide solutions to degradation during thermal treatment

Samples of isosorbide solutions with no antioxidant or with 100 ppm of antioxidants of table 2 prepared according to protocol of example 2 were submitted to the thermal treatment test according to the present application. Yellow colorations of each isosorbide solutions were measured before and after the thermal treatment test.

**[Table 2]**

| Sample # | Antioxidant compound | Yellow coloration index | | |
|---|---|---|---|---|
| | | before thermal test | after thermal test | After-Before |
| 1 | None | 1.2 | 5.3 | 4.1 |
| 2.1 | Benzophenone | 1.3 | 7.7 | 6.4 |
| 2.3 | 2-hydroxy-4-methoxybenzophenone | 2.2 | 6.1 | 3.9 |
| 2.2 | 4,4'-dihydroxy-benzophenone | 4.2 | 9.3 | 5.1 |
| 3 | Propyl gallate | 9.1 | 14.3 | 5.2 |
| 2.4 | 2,2'-dihydroxy-4-methoxybenzophenone | 12.3 | 49.3 | 37 |

In table 2, the values of yellow coloration index before thermal treatment are the result of the chemical structure of each antioxidant which define their inner yellow coloration. One can thus see that the substituents of the aromatic cycles induce an increased yellow coloration compared to benzophenone.

After thermal treatment, the yellow coloration indexes obtained are increased compared to before thermal treatment, because of the formation of yellowish compounds by thermal degradation of isosorbide and also of antioxidants.

The benzophenone advantageously gave the lowest yellow coloration index before thermal treatment, and a yellow coloration index after thermal treatment lower than 10, which corresponds to an un-noticeable yellow color at naked eyes. Somehow, the induction time of the solution of isosorbide containing benzophenone is only increased of about 9 % with respect to the solution of isosorbide free of antioxidant. Benzophenone is therefore mostly suitable for increasing the stability of isosorbide solutions against oxidation when the lowest yellow coloration index is needed and in uses where no thermal treatment of said isosorbide solution is to be carried out.

The 2,2'-dihydroxy-4-methoxy-benzophenone gave the highest coloration indexes both before and after thermal treatment test. Even though it allows the highest induction time according to the PetroOxy test, i.e. the highest protection against oxidation, the high yellow color might be troublesome for some of the end uses of isosorbide. This antioxidant thus can only be used for isosorbide solutions which will not undergo thermal treatments, for example such as the "thermal treatment test" of the present application, or for uses of isosorbide where the yellow color is acceptable or can be hidden by incorporating appropriate pigments into the final product containing isosorbide.

Compared to the 2,2'-dihydroxy-4-methoxy-benzophenone, the 4,4'-dihydroxy-benzophenone gave lower yellow coloration indexes, both before (three times lower) and after (almost five times lower) thermal treatment test, both values being lower than 10. Taking into account its induction time of 3.9 hours, this antioxidant offers a protection against oxidation equivalent to the benzophenone, but with higher yellow coloration indexes. It somehow represents an acceptable alternative to benzophenone.

Before thermal treatment test, the 2-hydroxy-4-methoxy-benzophenone surprisingly gave a six times lower yellow coloration than 2,2'-dihydroxy-4-methoxybenzophenone, even though the only structural difference is a missing hydroxyl group on one of the two aromatic cycles. It also gave the lowest yellow coloration after thermal treatment among all benzophenone derivatives tested, which amounts to a surprisingly four times lower increase of the yellow coloration after thermal treatment than 2,2'-dihydroxy-4-methoxy-benzophenone. It is unexpected that such a small difference in the chemical structure, i.e. the absence of hydroxy group in position 2 on one of the aromatic cyles, has a so big impact on the yellow coloration index. Since the 2-hydroxy-4-methoxy-benzophenone led to the second highest induction time according to PetroOxy test, and since it induces the lowest yellow coloration, this antioxidant is the best one of all those tested in table 1 and 2. It allows to maintain the yellow coloration index below 7 after thermal treatment, which corresponds to an un-noticeable yellow color at naked eyes, while offering a very good protection against oxidation. This antioxidant offers the best equilibrium between protection against oxidation and low yellow coloration.

## Claims

1. Composition comprising:
- at least one chemical compound having at least one heterocycle,
- and at least one antioxidant of formula (I): 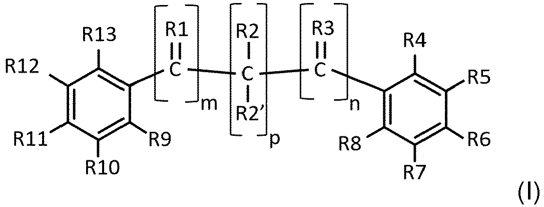
wherein:
- R1 and R3 are selected from oxygen, CH2, saturated or unsaturated alkyl groups comprising 2 to 10 carbon atoms, or a combination thereof,
- R2 and R2' are selected from hydrogen, CH3, saturated or unsaturated alkyl groups comprising 2 to 10 carbon atoms, aromatic cycles, or a combination thereof,
- R4 to R13 are selected from H, OH, CₓH₂ₓ₊₁, O-C_{y}H_{2y+1}, x and y being integers ranging from 1 to 10,
- m, n and p are integers in the range 0 to 10, and when p = 0 then m and/or n is greater than or equal to 1, or when both m and n are equal to 0 then p is greater than or equal to 1.

2. Composition according to claim 1, wherein the antioxidant is of formula (II): 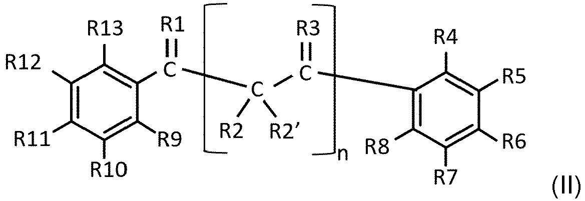 wherein:
- R1 and R3 are selected from oxygen, CH2, saturated or unsaturated alkyl groups comprising 2 to 10 carbon atoms, or a combination thereof,
- R2 and R2' are selected from hydrogen, CH3, saturated or unsaturated alkyl groups comprising 2 to 10 carbon atoms, aromatic cycles, or a combination thereof,
- R4 to R13 are selected from H, OH, CₓH₂ₓ₊₁, O-C_{y}H_{2y+1}, x and y being integers ranging from 1 to 10,
- n is an integer in the range 0 to 10.

3. Composition according to claim 1, wherein the antioxidant is of formula (III): 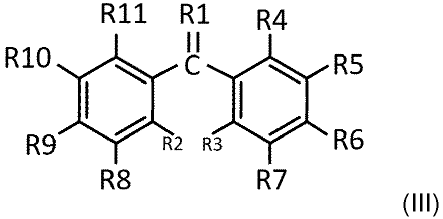 wherein:
- R1 is selected from oxygen, CH2, saturated or unsaturated alkyl groups comprising 2 to 10 carbon atoms,
- R2 to R11 are selected from H, OH, CₓH₂ₓ₊₁, O-C_{y}H_{2y+1}, x and y being integers ranging from 1 to 10.

4. Composition according to claim 3, wherein the antioxidant is benzophenone compound of formula (IV): 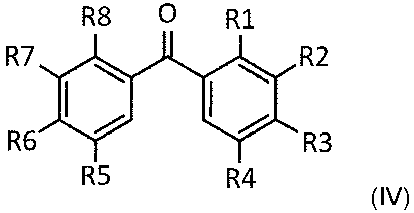 wherein R1 to R8 are selected from H, OH, CH3, O-CH3.

5. Composition according to claim 4, wherein the benzophenone compounds of formula (III) are compounds of formula (V) to (VIII):

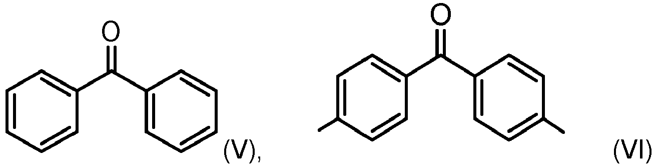

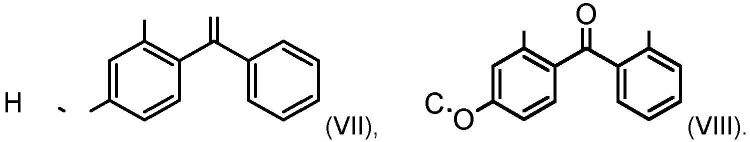

6. Composition according to claim 4, wherein the benzophenone compound of formula (III) is 2-hydroxy-4-methoxy benzophenone of formula (VII).

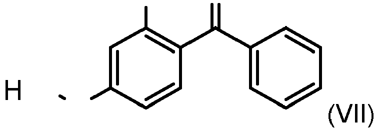

7. Composition according to any one of the preceding claims, wherein the content of the antioxidant in the composition is equal to or less than 1 000 ppm with respect to the weight of the organic chemical compound, in particular equal to or less than 500 ppm, or equal to or less than 400 ppm, or equal to or less than 300 ppm, or equal to or less than 200 ppm, or equal to or less than 100 ppm.

8. Composition according to any of the preceding claims, wherein the heterocyclic compound results from an intramolecular dehydration of a hydrogenated sugar,or is a derivative of a product of an intramolecular dehydration of a hydrogenated sugar.

9. Composition according to claim 8, wherein the hydrogenated sugar is selected from sorbitol, mannitol, or xylitol, or mixtures thereof, in particular sorbitol.

10. Composition according to any one of the preceding claims, wherein it exhibits an induction time greater than or equal to 3.8 hours, in particular greater than or equal to 4.0 hours, more particularly greater than or equal to 4.5 hours, most particularly greater than or equal to 5.0 hours, as measured according to the "PetroOxy test" at 120°C and 7 bars of oxygen.

11. Composition according to the preceding claim 10, wherein it exhibits a yellow coloration index equal to or less than 13.0 YID, in particular equal to or less than 5.0 YID, more particularly equal to or less than 3.0 YID, most particularly equal to or less than 2.5 YID.

12. Composition according to any of claims 10 or 11, wherein it exhibits a yellow coloration index after thermal treatment test, equal to or less than 50.0 YID, in particular equal to or less than 10.0 YID, more particularly equal to or less than 8.0 YID, most particularly equal to or less than 6.5 YID.

13. Process for preparing a composition as described in any of claims 1 to 12, comprising:
a) an intramolecular dehydration step of a hydrogenated sugar to obtain a dehydration product in a crude reaction mixture,
b) optionally a step of derivatization of the dehydration product obtained in step a) to obtain a derivative of the dehydration product in a crude reaction mixture,
c) optionally a step of distillation of the crude reaction mixtures obtained in step a) and/or in step b) to obtain a distillate enriched with the dehydration product or the derivative thereof,
d) optionally a step of purification of the crude reaction mixtures obtained in step a) and/or in step b), and/or of the distillate obtained in step c), to obtain a purified dehydration product,
e) optionally a step of shaping the crude reaction mixtures obtained in step a) and/or in step b), and/or shaping of the distillate obtained in step c), and/or shaping of the purified dehydration product obtained in step d), to obtain a shaped dehydration product,
f) a step of adding at least one antioxidant of formula (I) to the crude reaction mixtures obtained in step a) and/or in step b), and/or to the distillate obtained in step c), and/or to the purified dehydration product obtained in step d), and/or to the shaped dehydration product obtained in step e), 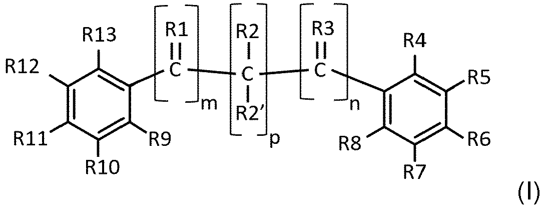 wherein:
- R1 and R3 are selected from oxygen, CH2, saturated or unsaturated alkyl groups comprising 2 to 10 carbon atoms, or a combination thereof,
- R2 and R2' are selected from hydrogen, CH3, saturated or unsaturated alkyl groups comprising 2 to 10 carbon atoms, aromatic cycles, or a combination thereof,
- R4 to R13 are selected from H, OH, CₓH₂ₓ₊₁, O-C_{y}H_{2y+1}, x and y being integers ranging from 1 to 10,
- m, n and p are integers in the range 0 to 10, and when p = 0 then m and/or n is greater than or equal to 1, or when both m and n are equal to 0 then p is greater than or equal to 1.

14. Use of an antioxidant of formula (I) 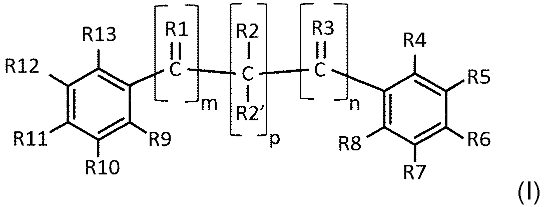 in a composition comprising at least one chemical compound having at least one heterocycle, for preventing or reducing the chemical degradation of said dehydration product or derivative thereof,
wherein:
- R1 and R3 are selected from oxygen, CH2, saturated or unsaturated alkyl groups comprising 2 to 10 carbon atoms, or a combination thereof,
- R2 and R2' are selected from hydrogen, CH3, saturated or unsaturated alkyl groups comprising 2 to 10 carbon atoms, aromatic cycles, or a combination thereof,
- R4 to R13 are selected from H, OH, CₓH₂ₓ₊₁, O-C_{y}H_{2y+1}, x and y being integers ranging from 1 to 10,
- m, n and p are integers in the range 0 to 10, and when p = 0 then m and/or n is greater than or equal to 1, or when both m and n are equal to 0 then p is greater than or equal to 1..

15. Use of a composition according to any one of claims 1 to 12 for the preparation of products or mixtures, polymeric or not, biodegradable or not, for the chemical, pharmaceutical, cosmetic or food industries.

16. Use according to claim 15, wherein the use is the preparation of polyesters or polycarbonates comprising said intramolecular dehydration product of a hydrogenated sugar as a co-monomer, in particular comprising isosorbide as a co-monomer.
